# EUROPEAN PATENT APPLICATION

(11) **EP 1 544 286 A1**
(43) Date of publication of application: **22.06.2005**
(21) Application number: 02758815.1
(22) Date of filing: 08.08.2002
(51) Int. Cl.: C12M 1/00, G01N 1/28

(54) **SINGLE CELL OPERATION SUPPORTING ROBOT**

(71) Applicant: National University Corporation Tokyo University of Agriculture and Technology, Fuchu-shi, Tokyo 183-8538 (JP)
(72) Inventor: MATSUOKA, Hideaki, Dept Biotech. and Life Science, Koganei-shi, Tokyo 184-8588 (JP); SAITO, Mikako, Dept Biotech. and Life Science, Koganei-shi, Tokyo 184-8588 (JP)
(74) Representative: Paget, Hugh Charles Edward
(86) International application number: PCT/JP2002/008139
(87) International publication number: WO 2004/015055

(57) **Abstract**

A single-cell operation supporting robot for supporting a single-cell operation work with multi-purposes by carrying out a work which an operator himself/herself carries out difficultly, according to the indication of the operator. The robot is equipped with a microscope 1, multi-micro well 4 for storing cells and a single-cell stimulating device 5 which are respectively provided on the stage 1a of the microscope 1, a sample injection transportation means 7 which transports a glass capillary 6 for injecting a sample into the single-cell relatively to and from the multi-microwell 4 and an automatic stage 2, a cell transportation device 9 which transports a glass capillary 8 for holding the single-cell relatively to and from each well of the multi-microwell 4, the single-cell stimulating device 5 and the glass capillary 6, a microscopic spectral measurement device 10 which is combined with the microscope 1, computers 11 and 12 which automatically control an actuation of at least one among the microscope 1, the sample injection transportation device 7, the cell transportation device 9, the automatic stage 2, the single-cell stimulating device 5 and the microscopic spectral measurement device 10, and a manual operation device 17 which inputs signals to the computers 11 and 12 based on an operation by an operator and actuates the microscope and the like.

## Description

### TECHNICAL FIELD

The invention relates to a robot that support the operation of animal and plant single-cells with diameters of ten and several µm or greater.

### BACKGROUND OF THE INVENTION

Formerly many of a series of operations consisting of the holding, transportation, and arrangement of single-cells on a microscope stage, the injection of various samples such as genes and drugs into each single-cell, the application of stimulations (electric, thermal, mechanical, and/or electrochemical stimulations) to each single-cell, and the measurement of cell responses and the dynamic behavior of cellular molecules, required delicate and well-trained techniques and therefore it was difficult for an operator to carry out these operations for himself/herself. Further, since it was conventionally difficult to treat many single-cells by individually discriminating them, the operator had to operate the single-cells as a group, or treat the single-cells as a group without individually discriminating them even after the respective single-cells were operated one by one.

Accordingly, the object of the present invention is to provide a robot for supporting single-cell operation works with multi-purposes by carrying out works which an operator can hardly carry out for himself/herself, according to the indication of the operator. Further, the object of the present invention is to provide a robot which enables the discrimination of each single-cell throughout by numbering respective cells.

### DISCLOSURE OF THE INVENTION

The single-cell operation supporting robot of the present invention which achieves the above-mentioned purpose comprises,
(1) a microscope;
(2) a microwell for storing cells on the microscope stage;
(3) a single-cell stimulating device on the microscope stage;
(4) a sample injection device on the microscope stage;
(5) a single-cell holding device on the microscope stage;
(6) a means to transport (4) relatively to and from (2);
(7) a means to transport (5) relatively to and from (2), (3), (4);
(8) a single-cell measuring device combined with (1);
(9) at least one computer which automatically controls the actuation of at least one among (1), (6), (7), (3), (8), according to a program installed beforehand; and
(10) a means for the manual operation so that an operator can input signals to the computer to control at least one among (1), (6), (7), (3), (8).

This single-cell operation supporting robot can be operated either by automatic or manual operation mode. In the automatic operation mode, at least one computer automatically controls the actuation of at least one among (1), (6), (7), (3), (8), according to a computer program which is installed beforehand. In the manual operation mode, an operator can input signals to the computer to control at least one among (1), (6), (7), (3), (8).

The single-cell operation supporting robot of the present invention can operate the following works which need to be carried out at high speed with high positional precision; the holding and transportation of a single-cell; the transportation of the sample injection device to a position close to the single-cell; the change of the magnification of objective lens during those works; the single-cell stimulation; and the measurement with a single-cell measuring device such as a microscopic spectrophotometer.
If necessary, some or all of these works can be automated and the load of an operator can be greatly reduced; and therefore, the operator can concentrate his/her attention on such works that requires utmost carefulness as the microinjection and the analytical judgment on the measured results and the like.

Further, in the single-cell operation supporting robot of the present invention, the microwell for storing cells may preferably be such a multi-microwell that a plural number of wells are arranged regularly at the fixed addresses and each well can be used as a proprietary well for one single-cell. It is because cell operation requiring fine conditional settings can be carried out if the plural number of wells can be used as respectively used as proprietary wells for respective single-cells throughout a series of cell operation works and a plural number of single-cells can be discriminated by the number of wells in which each single-cell is stored.

Further, in the single-cell operation supporting robot of the present invention, a means for the transportation of a sample injection device may preferably have an automatic stage which is provided on the microscope stage and which can transport a microwell for storing cells and a single-cell stimulating device relatively to and from the test sample injection device. It is because the moving range of the means for the transportation of the sample injection device can be narrowed and consequently its interference with the cell transportation means can be easily prevented.

Further, in the single-cell operation supporting robot of the present invention, a means for the transportation of the test sample injection device may preferably have a manipulator which can transport the sample injection device relatively to and from the stage, because it enables finer injection operation.

Further, in the single-cell operation supporting robot of the present invention, a means for the transportation of a cell holding device may preferably have an automatic stage which is provided on the microscope stage and which can transport a microwell for storing cells and a single-cell stimulating device relatively to and from the stage, because the moving range of the cell transportation means can be narrowed and consequently its interference with the means for the transportation of the sample injection device can be easily prevented. Further, if this automatic stage is used in combination with the means for the transportation of a sample injection device, a mechanism for transportation can be simplified and it is more preferable from the viewpoint of space efficiency and production cost.

Further, in the single-cell operation supporting robot of the present invention, the cell transportation means may preferably have a manipulator which can transport the cell holding means relatively to and from the stage, because it enables finer cell transportation operation.

### BRIEF DESCRIPTION OF THE DRAWINGS

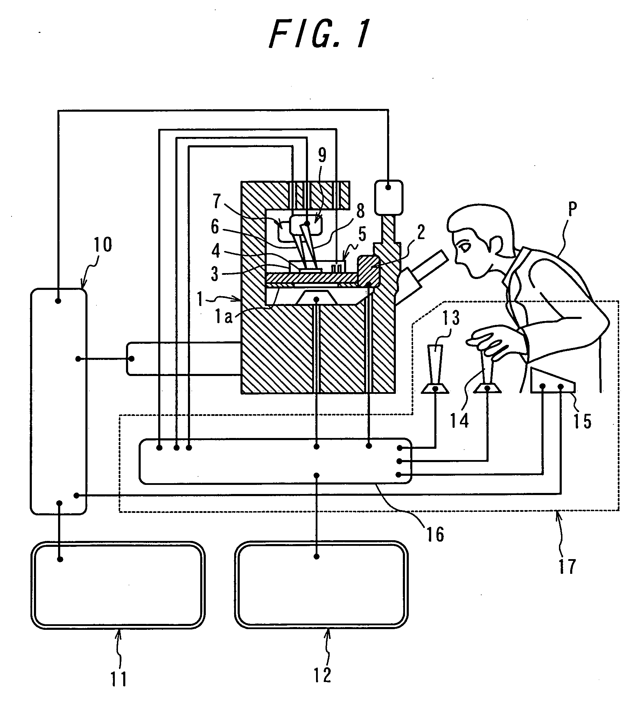

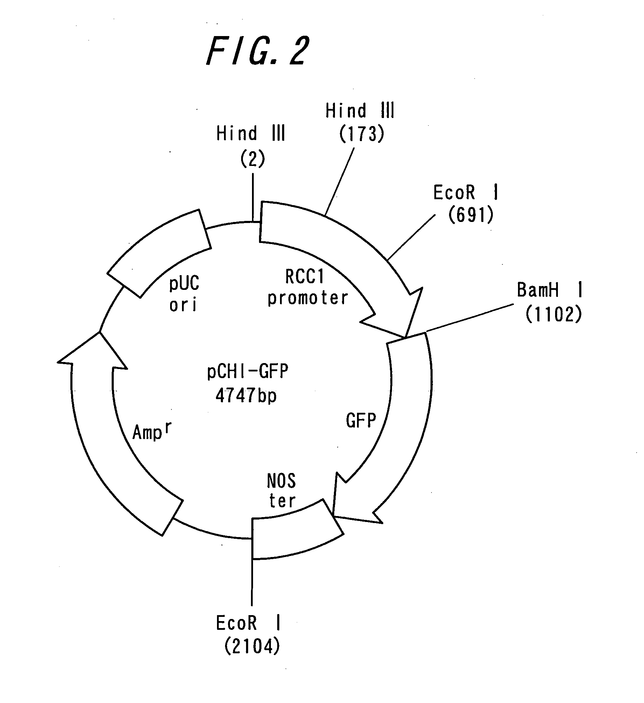

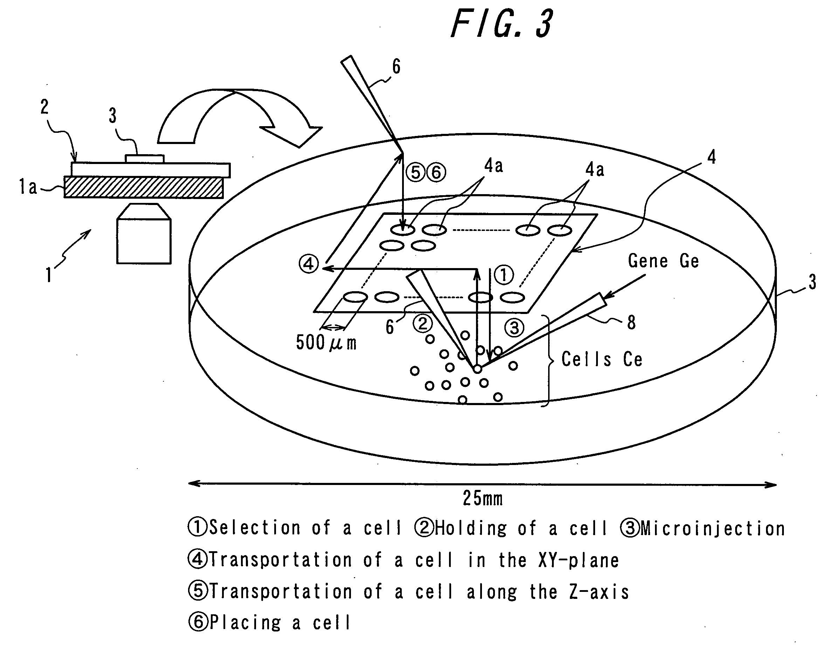

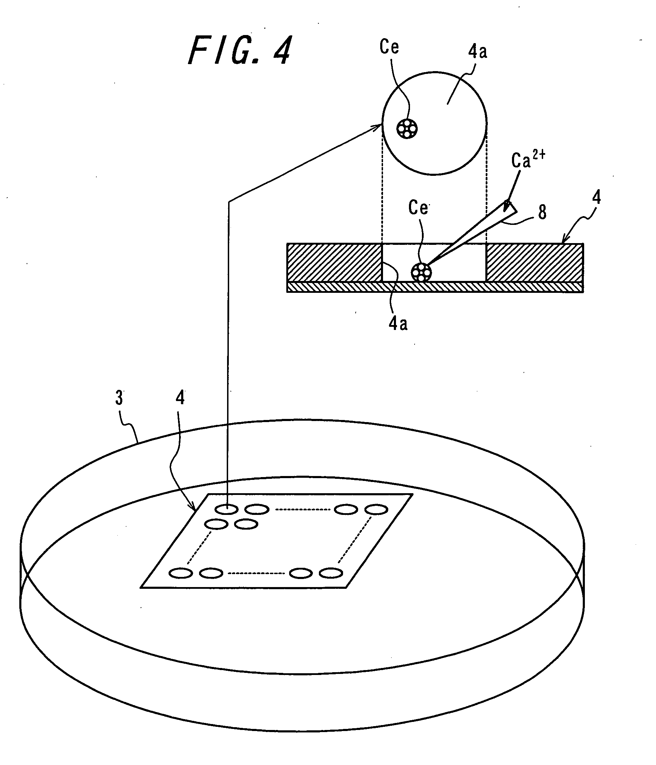

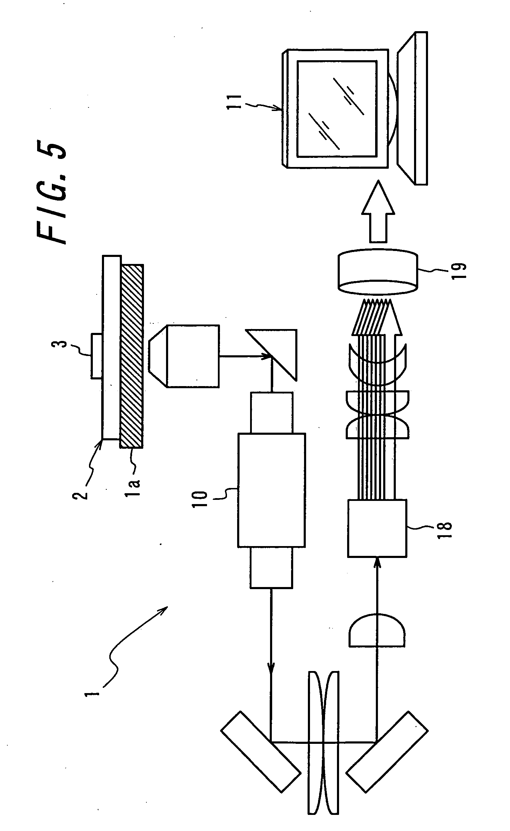

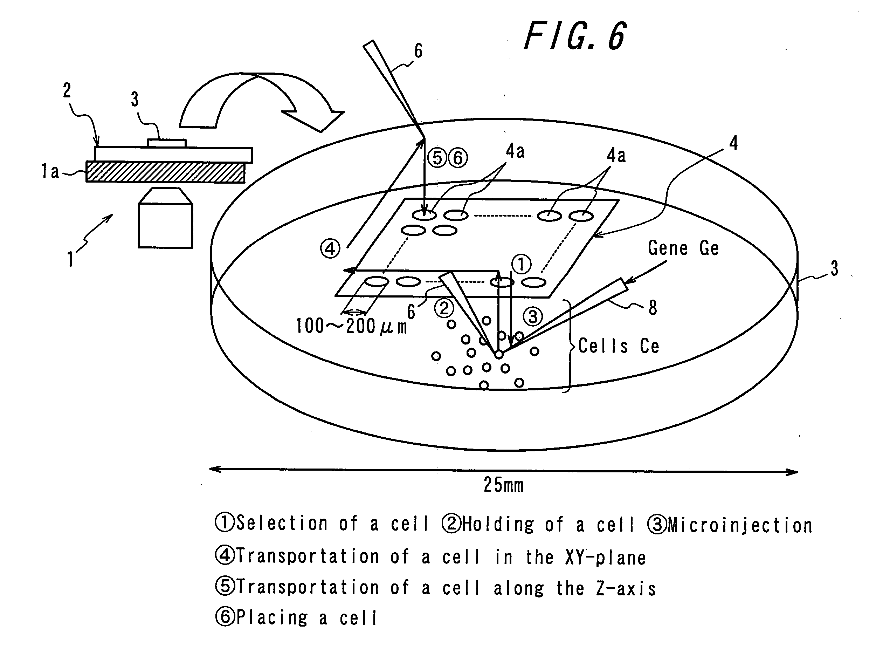

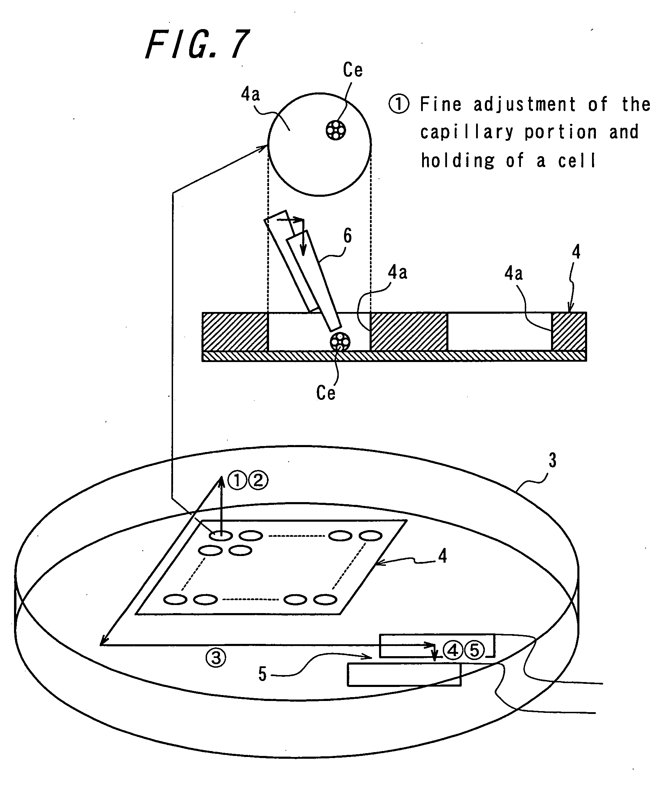

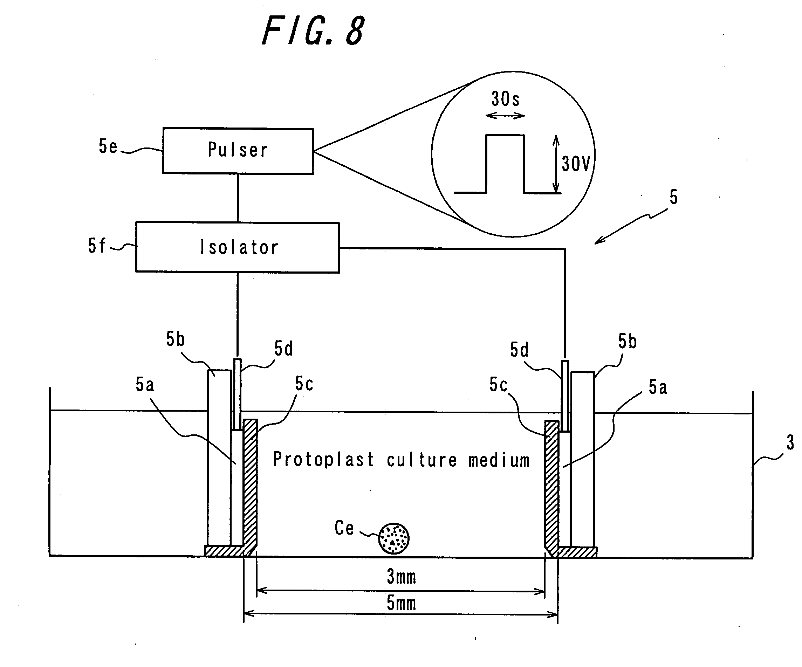

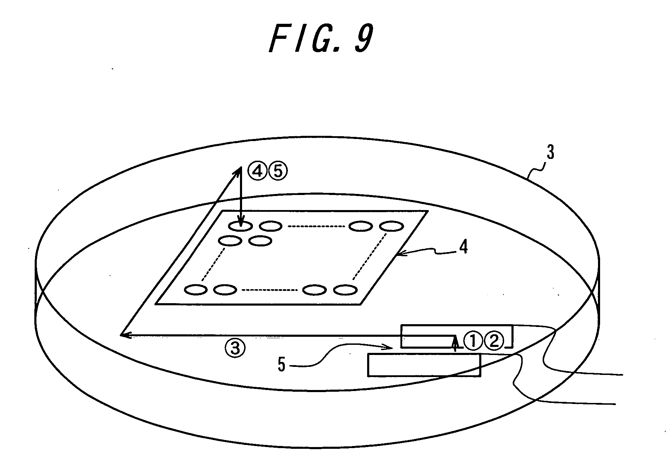

Fig.1 is a composition diagram schematically showing one Example of the single-cell operation supporting robot of the present invention. Fig.2 is an illustration diagram showing a plasmid which was used for the experiment of a gene expression using the single-cell operation supporting robot of the above-mentioned Example. Fig.3 is an illustration diagram showing the operation of injecting a gene in the experiment of the gene expression using the single-cell operation supporting robot of the Example. Fig.4 is an illustration diagram showing the operation of injecting Ca²⁺ in the experiment of the gene expression using the single-cell operation supporting robot of the Example. Fig.5 is an illustration diagram showing the method of microscopic fluorescence measurement in the experiment of the gene expression using the single-cell operation supporting robot of the Example. Fig.6 is an illustration diagram showing the operation of injecting another gene in the experiment of another gene expression using the single-cell operation supporting robot of the Example. Fig.7 is an illustration diagram showing the operation of applying an electrical stimulation in the experiment of the second gene expression using the single-cell operation supporting robot of the Example. Fig.8 is an illustration diagram showing the composition of the single-cell stimulation device in the single-cell operation supporting robot of the Example. Further, Fig.9 is an illustration diagram showing the operation after applying electrical stimulation in the experiment of the second gene expression using the single-cell operation supporting robot of the Example.

### BEST MODE FOR PERFORMING THE INVENTION

The embodiments of the present invention are specifically illustrated below according to Examples based on the drawings. Herein, Fig.1 is a composition diagram showing one Example of the single-cell operation supporting robot of the present invention. As shown in the illustration, the single-cell operation supporting robot of the Example is equipped with a microscope 1, multi-microwells 4 for storing cells as the microwells for storing cells which were respectively provided in culture dishes 3 fixed on an automatic stage 2 which was provided on the stage 1a of the microscope 1 and a single-cell stimulation device 5, a sample injection transfer means 7 which composes the sample injection transfer means which transports a glass capillary 6 as a sample injection means for injecting a sample into the single-cell, relatively to and from the multi-microwells 4 for storing cells, a cell transportation device 9 which composes the cell transportation means which transports an another glass capillary 8 as a cell holding means for holding the single-cell, relatively to and from each of the multi-microwells 4 for storing cells, the single-cell stimulation device 5 and the previous glass capillary 6, a microscope spectrophotometric measurement device 10 as a single-cell measuring device which was combined with the microscope 1, and the automatic stage 2 which is used in combination with the sample injection device transportation means and the cell transportation means and composes these transportation means.

Further, the single-cell operation supporting robot of the Example is equipped with a personal computer (PC) 11 which automatically controls the actuation of at least one among the microscope 1 and the microscope spectrophotometer 10 based on a program which was installed beforehand, a personal computer (PC) 12 which automatically controls the actuation of at least one among the single-cell stimulation device 5, the sample injection transportation device 7, the cell transportation device 9 and the automatic stage 2 based on a program which was installed beforehand, and a manual operation device 17 having two joystick type controllers 13 and 14, a keyboard 15 and an interface 16 as a manual operation means which inputs signals to the PCs 11 and 12 based on an operation by an operator and actuates at least one of the microscope 1, the single-cell stimulation device 5, the sample injection transportation device 7, the cell transportation device 9, the microscope spectrophotometric measurement device 10 and the automatic stage 2 by corresponding it to the above-mentioned operation. Further, the operator P can control the actuations of the automatic stage 2, the sample injection transportation device 7 and the cell transportation device 9 by operating the two joystick type controllers 13 and 14 while viewing the microscope 1 or while viewing the monitor image of a microscope image. Then, the PC's 11 and 12 carry out calculation and analysis which are necessary for the inspection of the above-mentioned respective elements through the interface 16, automatic control and manual control, the recording of calculated data and image processing and the like.

In the single-cell operation supporting robot of the Example, an inverted fluorescent microscope IMT-2 equipped with an epifluorescence device (an exciting light irradiation device) manufactured by Olympus Optics Co., Ltd. is used as the microscope 1. Further, the automatic stage 2 composed of a usual double axial orthogonal coordinate transportation table (double axial electromotive control) manufactured by Chuo Precision Industrial Co., Ltd. Further, the sample injection transportation device 7 supports a uniaxial linear actuating micromanipulator (manually operating hydraulically actuated) manufactured by Narishige Co. with a manipulator with three variances (x, y and z axes, electromotive control of the three variances within an angle θ versus an xy-plane) manufactured by Eppendorf AG., and supports a usual injection holder with the uniaxial linear actuating manipulator. The glass capillary 6 which injects a sample into the single-cell is installed on the injection holder and can be moved at the four variances.

Further, in the single-cell operation supporting robot of the Example, the cell transportation device 9 supports the uniaxial linear actuating micromanipulator with a usual triple axial orthogonal coordinate manipulator and supports a usual injection holder with the uniaxial linear actuating manipulator. The glass capillary 8 which holds the single-cell is installed on the injection holder and can be moved at the four variances.

Further, in the single-cell operation supporting robot of the Example, the multi-microwell 4 for storing cells has wells 4a which are concaves with a diameter of about 500 µm for storing the single-cell were formed at 100 pieces in total by 5 longitudinal pieces x 10 lateral pieces at a fixed interval at two fixed positions on the surface of a vinyl chloride plate, in order to carry out the storage of one cell in intra-well solution, the operation of applying stimulation in wells, the optical, electrical, electrochemical and physical measurements of cells, transportation from the wells to other places and the like, in the sight of the microscope. Further, the single-cell stimulation device 5 is a minute device which applies an electrical, thermal, mechanical or chemical stimulation to a cell which was taken out from the multi-microwell for storing cells, at other place in the sight of the microscope. In the example shown in Fig.1, two of Pt (platinum) plate electrodes 5a are respectively sandwiched by an acryl plate 5b and a dialysis membrane 5c, and Pt lead wires 5d are provided to the respective Pt plate electrodes 5a, as shown in Fig.8. Further, herein, the sample injection transportation device 7 is also inserted in the multi-microwell for storing cells, is also a minute device which applies an electrical, thermal, mechanical or chemical stimulation to a cell which was taken out from the multi-microwell for storing cells, at other place in the sight of the microscope and composes the single-cell stimulation device.

Further, in the single-cell operation supporting robot of the Example, there is a spectrophotometric-imaging device which has performances that the highest space resolution is 1 µm × 1 µm, the highest wavelength resolution is 5 nm within a range from 400 nm to 800 nm and a time resolution can record one image per 5 seconds and which was developed by Tokyo University of Agriculture and Technology and Genesia Corporation and let already known, as the microscope spectrophotometer 10.

Then, the introduction of a gene into a rice single-cell and the experiment of gene expression by stimulation of introducing Ca²⁺ using the single-cell operation supporting robot of the Example are illustrated.

### (Cell culture)

Rice cells (*Oryza sativa* L. japonica cv. Nipponbare) were cultured and the calli were filtered through a metal net of 1.0 mm mesh to be used. 27 ml of a specific medium was charged in a 100 ml flask and rotary incubation (100 rpm, 25°C, dark place) was carried out. The culture was maintained by a regular transfer of 7-day-old calli. After the last transfer, the 4-day-old calli were collected to be used for the experiment below. (Preparation of plasmid (plasmid is a cyclic DNA and a gene can be incorporated in the ring))

A plasmid 35S-GFP which was purchased from Clontech Laboratories, Inc., contains a promoter which is called as "35S" that enables the expression of any gene at any time in plant cells, and a gene of the green fluorescent protein (GFP). Accordingly, when this is introduced in a plant cell, GFP is generated in the cell and green fluorescence is emitted.

The portion of 35S promoter of the plasmid was permutated with the promoter of rice chitinase (CHI) gene. Thus, pCHI-GFP as shown in Fig. 2 was prepared and used in the experiment thereafter. When an elicitor (oligosaccharide having a chitin configuration which is a constitutional component of the cell wall of fungus) is applied to the rice cell, the chitinase gene should be originally expressed, but since the gene of GFP is incorporated in place of chitinase, the information of "the expression of chitinase gene" can be visualized by the generation of "GFP". This is measured by fluorescent microscopy.

### (Preparation of rice protoplast)

Protoplasts were prepared using 4-day-old calli of rice. Namely, the calli were separated from the supernatant and washed with a rinsing solution (0.4 M mannitol solution). To this, 10 ml of an enzyme solution was added, and the mixture was shaken at 30°C and 30 rpm for 1 hr in the dark, and then it was stood still for successive 2 hr at 30°C. After that, the callus suspension was filtered through a nylon net with 40 µmφ mesh to collect protoplasts in a 50 ml centrifugal tube. The tube was centrifuged at 1000 rpm for 60 sec to collect the protoplasts as the precipitate.
The supernatant containing enzyme was discarded, and 5 ml of the protoplast medium was added to suspend protoplasts. The protoplasts are such cells that the cell wall has been removed and they are merely described as "cells" hereinafter.

Further, the compositions of the above-mentioned rinsing solution and the enzyme solution are as follows.

| | | |
|---|---|---|
| a. | Rinsing solution (0.4 M mannitol solution) | |
| | Mannitol | 36.43 g |
| | Distilled water | 500 ml |
| b. | Enzyme solution (pH 5.6) | |
| | Pectolyase Y-23 | 0.05 g |
| | Cellulase Onozuka RS | 2.0 g |
| | CaCl₂-2H₂O | 0.23 ml |
| | K Dexstran sulfate | 0.1 g |
| | Mannitol | 9.0 g |
| | Distilled water | 200 ml |

### (Preparation of a glass capillary 6 used for the holding and transportation of cells)

A single channel glass capillary (a diameter of 1 mm) (GD-1, manufactured by Narishige Co., Ltd.) was rinsed with ethanol, and sterilized by dry heating at 120°C for 3 hr. This was pulled with a two step-pulling type puller (PP83, manufactured by Narishige Co., Ltd.) and the edge was rounded with a microforge (MF-83, manufactured by Narishige Co., Ltd.). Finally, the edge of the capillary was set as an outer diameter of about 50 µm and an inner diameter of about 20 µm.

### (Preparation of a glass capillary 8 used for injection into cells)

A glass capillary sterilized was pulled with a laser puller (P-2000, SUTTER INSTRUMENT Co.) and the diameter of edge was set so as to be 1 µm or less.

### (Injection of plasmid)

As shown in Fig.3, firstly, a plasmid 35S-GFP solution which was prepared at 1.0 µg·µl⁻¹ was introduced in a glass capillary 8 for injection into cells. The glass capillary 8 was held with the capillary holder and connected with a pico pump, and the gene Ge of GFP was designed to be injected by applying the pressure of nitrogen gas. On the other hand, the glass capillary 6 for holding cells was fixed with the capillary holder, and the inside was filled with purified water to be connected with the injector, and the cells Ce was designed to be held by suction with the injector.

Then, a culture dish 3 in which the multi-microwell 4 was installed was mounted on the stage 1a of a microscope 1. As shown in (1) in Fig.3, one of the cells Ce (a diameter of about 20 to about 30 µm) which were dispersed at the bottom of the dish 3 was selected, and as shown in (2) in Fig.3, this was held by suction with the glass capillary 6 for holding cells. As shown in (3) in Fig.3, the glass capillary 8 for injection into cells was injected in the protoplasm of the cell Ce using the means for the transportation of the sample injection device 7, and the gene Ge was injected into the cell by applying pressure in the glass capillary 8 with a pico pump. The cell Ce to which the gene injection (micro injection) was carried out was transported by the automatic stage 2 and the cell transportation device 9 which were automatically controlled by PC 12 as shown in (4) and (5) in Fig.3 and stored in the well 4a of the fixed positional number of the multi-microwell 4, to define the cell number. Thus, after completing the micro injection and operation of storing cells for 50 to 100 cells, those cells Ce were stood still for the incubation at 25°C for 24 hr as shown in (6) in Fig.3.

### (Injection of Ca²⁺ into cells)

The cells Ce adhered at the bottom of the wells by incubation for 24 hr. As shown in Fig.4, the glass capillary 8 for injection into cells filled with CaCl₂ (1 µM, 10 µM or 100 µM) was injected into the cells Ce using the means for the transportation of the sample injection device 7 as shown in Fig.1 and Ca²⁺ was injected. After successive incubation at 25°C for 24 hr, the cells Ce were photographed using the microscope 1 and the microscopic spectral analysis device 10 as shown in Fig.5, and the presence or absence of the generation of GFP was studied by microscopic fluorescence measurement. Here, a code 18 in the drawing shows a grism and the code 19 shows a CCD camera.

### (Measurement of the expression of chitinase gene)

The frequencies of the chitinase gene expression caused by the injection of Ca²⁺ were 18% (9 out of 50 cells), 12% (6 out of 50 cells) and 0% (zero out of 50 cells), when the concentrations of CaCl₂ were 1 µM, 10 µM and 100 µM, respectively.

Finally, the procedures for the introduction of a gene into a rice single-cell and the experiment of gene expression by electrical stimulation using the single-cell operation supporting robot of the Example are illustrated. The incubation of the cells, the preparation of plasmid, the preparation of rice protoplasts, the preparation of the glass capillary 6 for holding and transporting cells and the preparation of the glass capillary 8 for injection into cells are same as the previous experiments, and their illustrations are omitted.

### (Injection of plasmid)

As shown in Fig.6, the experiment is same as the previous experiment except that the cells Ce are placed in the multi-microwell 4 from the beginning.

### (Introduction of Fura 2 in cells)

Fura 2-AM (DOJINDO) which was dissolved in DMSO was applied to the cells which were incubated in the multi-microwell 4 so as to be a final concentration at 4 µM and the cells were stood still for 1 hr at 30°C in the dark so that Fura2-AM is to be incorporated in the cells. Then, the external solution of the cells was replaced by a fresh medium, and the experiment of electrical stimulation described below was carried out.

### (Application of electrical stimulation)

As shown in (1) in Fig.7, the cells Ce in the multi-microwell 4 were held with the glass capillary 6 for holding, and transported along the route, as shown in (2) to (4) in Fig.7, to the midpoint between Pt plate electrodes 5a of the single-cell stimulating device 5, as shown in Fig.8, and placed there alone as shown in (5) in Fig.7. Then a direct current pulse with a pulse height of 30V was applied for 30 sec from a pulse 5e between the Pt plate electrodes 5a via an isolator 5f and Pt lead wires 5d. After the application of the electrical stimulation, the cells Ce were stored again in the original wells 4a as shown in (1) to (5) in Fig.9 and incubated at 25°C for 24 hr.

### (Measurement of expression of chitinase gene)

100 Cells were studied by microscopic fluorescence measurement in the same manner as the previous experiment. There were 6 cells in which the transient increase of the Ca²⁺ concentration in cell (it reached the peek around 25 sec and returned to its original level after 50 sec) by the electrical stimulation was observed. The gene expression was observed in all these cells. There was another case that two cells showed a gradually increasing pattern (it reached the peek around 100 sec and gradually decreased thereafter but it did not return to its original level). In this case, the gene expression was also observed in all these cells. On the other hand, the gene expression was not observed in the other cells in which the change of the concentration of Ca²⁺ was not observed. Among them, however, 6 cells showed gene expression when elicitor was finally applied to the cells. These results indicate that the expression of chitinase gene can be induced by the electrical stimulation via Ca²⁺ influx in cells.

Thus, the single-cell operation supporting robot of the Example enables the holding and transportation of the single-cell Ce, the transportation of the glass capillary 8 for injection into cells to a position close to the single-cell Ce, the stimulation of the single-cell Ce, which require a high positional precision and high transportation speed. The successive measurement with the single-cell measuring device such as a microscopic spectrophotometer, and the like, also can be done automatically.
If necessary, some or all of those operations can be automated and the load of the operator can be greatly reduced; and therefore, the operator can concentrate his/her attention on the injection of a sample (a gene and Ca²⁺) into the single-cell which requires particular carefulness, and on the analytical judgment on the measured results and the like.

Further, according to the single-cell operation supporting robot of the Example, the microwell for storing cells is a multi-microwell 4 in which a plural number of wells 4a which can be respectively used as a proprietary well for one single-cell are arranged at a fixed arrangement, and respective wells 4a can be respectively used as a proprietary well for one single-cell throughout a series of cell operations. Therefore a plural number of cells can be discriminated by the number of wells 4a in which each single-cell is stored, and cell operations with finer condition setting can be carried out.

Further, according to the single-cell operation supporting robot of the Example, a means for the transportation of the sample injection is provided on the stage 1a of the microscope 1 and has the automatic stage 2 which transport the multi-microwell 4 for storing cells and the single-cell stimulating device 5 relatively to and from the stage 1a. Therefore, the transportation range of the glass capillary 8 for injection into cells by the sample injection transportation device 7 can be narrowed and the interference with the cell transportation device 9 can be easily prevented.

Further, according to the single-cell operation supporting robot of the Example, the sample injection transportation device 7 which composes the sample injection transportation means has a manipulator which transport the glass capillary 8 for injection into cells relatively to and from the stage 1a. Therefore, finer injection operation can be carried out.

Further, according to the single-cell operation supporting robot of the Example, the cell transportation means is provided on the stage 1a of the microscope 1 and has the automatic stage 2 which transports the multi-microwell 4 for storing cells and the single-cell stimulating device 5 relatively to and from the stage 1a. Therefore, the transportation range of the glass capillary 6 for cell holding by the cell transportation device 9 can be narrowed and the interference with the means for the transportation of the sample injection device 7 can be easily prevented. Further, since the cell transportation means uses the same automatic stage 2 with the means for the transportation of the sample injection device, a mechanism for transportation can be simplified, and space efficiency can be enhanced and production cost can be reduced.

Then, according to the single-cell operation supporting robot of the Example, since the cell transportation device 9 which composes the cell transportation means has a manipulator which transports the glass capillary 6 for cell holding relatively to and from the stage 1a, finer transportation operation can be carried out.

The present invention is illustrated based on examples shown in the drawings above, but the present invention is not limited to the above-mentioned example. For example, the means for the transportation of the sample injection device and the cell transportation means may be composed of only a manipulator without the automatic stage 2 and the operation of the injector injecting a sample may be also carried out by an electromagnetic solenoid and the like and automated by computer control. Further, the manual operation means may have a pushing button switch, a stepping-on switch and the like. Further, the microscope 1 may be provided with the electromotive switch for the magnification change of the objective lens either by automatic control by a personal computer 11 or by manual control. Further, other devices than the microscope spectroscopic measurement device may be used for the single-cell measurement.

### INDUSTRIAL APPLICABILITY

According to the single-cell operation supporting robot of the present invention, the holding and transportation of the single-cell, the transportation of the sample injection means to a position close to the single-cell, the magnification change of the microscope between those works and the successive measurement with the single-cell measuring device such as a microscopic spectrophotometer, which require high positional precision and rapid transportation speed, can be automatically carried out. If necessary, some or all of those operations can be automated and the load of the operator can be greatly reduced; and therefore, the operator can concentrate his/her attention on the injection of a sample into the single-cell which requires particular carefulness, and on the analytical judgment on the measured results and the like.

## Claims

1. A single-cell operation supporting robot, comprising:
a microscope;
a microwell for storing cells and a single-cell stimulating device which are respectively provided on the stage of the microscope;
a means for the transportation of a sample injection device which transports a sample injection means for injecting a sample into a single-cell relatively to and from the microwell for storing cells;
a cell transportation means which transports a cell holding means for holding a single-cell relatively to and from each well of the microwell for storing cells, the single-cell stimulating device and the sample injection means;
a single-cell measuring device which is combined with the microscope;
at least one computer which automatically controls an actuation of at least one among the microscope, the sample injection transportation means, the cell transportation means, the single-cell stimulating device and the single-cell measuring device based on a program installed beforehand; and
a manual operation means which inputs signals to the computer based on an operation by an operator and actuates at least one of the microscope, the sample injection transportation means, the cell transportation means, the single-cell stimulating device and the single-cell measuring device.

2. The single-cell operation supporting robot according to Claim 1, wherein the microwell for storing cells is a multi-microwell in which a plural number of wells which can be respectively used as a proprietary well for one single-cell are arranged at a fixed arrangement.

3. The single-cell operation supporting robot according to Claim 1, wherein the sample injection transportation means has an automatic stage which is provided on the stage of the microscope and which transports the microwell for storing cells and the single-cell stimulating device relatively to and from the stage.

4. The single-cell operation supporting robot according to Claim 1, wherein the sample injection transportation means has a manipulator which transports the sample injection means relatively to and from the stage.

5. The single-cell operation supporting robot according to Claim 1, wherein the cell transportation means has an automatic stage which is provided on the stage of the microscope and which transports the microwell for storing cells and the single-cell stimulating device relatively to and from the stage.

6. The single-cell operation supporting robot according to Claim 1, wherein the cell transportation means has a manipulator which transports the cell holding means relatively to and from the stage.
